(19) Europäisches Patentamt / European Patent Office / Office européen des brevets

(11) **EP 3 970 624 B1**

(12) **EUROPEAN PATENT SPECIFICATION**

(45) Date of publication and mention
of the grant of the patent:
**19.07.2023 Bulletin 2023/29**

(21) Application number: **20826453.1**

(22) Date of filing: **19.06.2020**

(51) International Patent Classification (IPC):
*A61B 3/00* *(2006.01)*    *A61B 3/113* *(2006.01)*
*A61B 5/00* *(2006.01)*    *A61B 5/16* *(2006.01)*

(52) Cooperative Patent Classification (CPC):
**A61B 3/113; A61B 5/163; A61B 5/4088**

(86) International application number:
**PCT/JP2020/024264**

(87) International publication number:
**WO 2020/256148 (24.12.2020 Gazette 2020/52)**

(54) **EVALUATION DEVICE, EVALUATION METHOD, AND EVALUATION PROGRAM**

BEURTEILUNGSVORRICHTUNG, BEURTEILUNGSVERFAHREN UND
BEURTEILUNGSPROGRAMM

DISPOSITIF D'ÉVALUATION, PROCÉDÉ D'ÉVALUATION ET PROGRAMME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.06.2019 JP 2019114539**

(43) Date of publication of application:
**23.03.2022 Bulletin 2022/12**

(73) Proprietor: **JVCKenwood Corporation
Yokohama-shi, Kanagawa 2210022 (JP)**

(72) Inventors:
• **SHUDO, Katsuyuki
Yokohama-shi, Kanagawa 221-0022 (JP)**

• **KITO, Makoto
Yokohama-shi, Kanagawa 221-0022 (JP)**

(74) Representative: **Schmidbauer, Andreas Konrad
Wagner & Geyer Partnerschaft mbB
Patent- und Rechtsanwälte
Gewürzmühlstrasse 5
80538 München (DE)**

(56) References cited:
**WO-A1-2016/022414    WO-A1-2019/098173
WO-A1-2019/098173    CN-A- 108 495 584
JP-A- 2017 158 866    JP-A- 2017 217 051
JP-A- 2018 140 007    JP-A- 2018 140 007**

**Description**

Field

The present disclosure relates to an evaluation device, an evaluation method, and an evaluation program.

Background

In recent years, cognitive dysfunction and brain dysfunction are considered to be on the rise and it is required to find such cognitive dysfunction and brain dysfunction at early stages and quantitatively evaluate severity of symptoms. A method of, when making an evaluation on, for example, dementia with Lewy bodies among such cognitive dysfunction and brain dysfunction, asking a subject whether the subject has visual hallucinations and calling on the subject to answer has been disclosed (for example, refer to JP 2017-217051 A).

WO 2019 098173 A discloses a cognitive dysfunction diagnostic apparatus which is provided with: a display unit which displays an image for diagnosis of cognitive dysfunction on a display surface; an imaging unit which captures images of a patient's eye; a detection unit which detects the patient's viewpoints within the display surface in a time-sequence manner on the basis of the images captured by the imaging unit; a creation unit which creates a distribution map representing a distribution of the viewpoints detected by the detection unit; a storage unit which stores case characteristic data indicative of characteristics of a viewpoint distribution that corresponds to a typical case of cognitive dysfunction; and a diagnosis unit which provides a diagnostic of the patient's cognitive dysfunction by determining whether or not the distribution map shows characteristics of the case characteristic data.

JP 2017 158866 A discloses a diagnosis support device which includes: an image data acquisition part for acquiring image data of an eye ball of a subject; a fixation point detection part for detecting position data of a fixation point of the subject; a display control part for displaying a pattern having a feature point and being formed by at least one of a straight line and a curve on a display part; an area setting part for setting a specific area including the feature point on a display screen of the display part; a determination part for determining whether or not, the fixation point exists in the specific area based on position data of the fixation point; a calculation part for calculating time data indicating an existence time when the fixation point existed in the specific area, based on determination data of the determination part; and an output control part for outputting the time data

JP 2018 140007 A discloses an evaluation device which includes: an image data acquisition part for acquiring image data on a subject's eyeball; a fixation point detection part for detecting position data on the subject's fixation point based on the image data; a display control part for causing a plurality of target videos to be displayed in a display screen; a region setting part for setting a specific region corresponding to each of the plurality of target videos in the display screen; a determination part for determining whether or not the fixation point is present in each specific region based on the position data on the fixation point, and outputting determination data; an arithmetic part for obtaining region data indicating the specific region where the fixation point is first detected of the plurality of specific regions based on the determination data; an evaluation part for obtaining evaluation data on the subject based on the region data; and an output control part for outputting the evaluation data.

Summary

Technical Problem

The method described in JP 2017-217051 A, however, depends on a subjective view of a subject and thus is with low objectivity and it is difficult to obtain accurate evaluation.

The disclosure was made in view of the above-described circumstances and an object of the disclosure is to provide an evaluation device, an evaluation method, and an evaluation program that make it possible to make an evaluation on cognitive dysfunction and brain dysfunction accurately.

Solution to Problem

In accordance with the present invention, an evaluation device and evaluation method as set forth in the appended claims is provided. In particular, an evaluation device according to the present disclosure comprising: a display unit; a point-of-gaze detector configured to detect a position of a point of gaze of a subject on the display unit; a display controller configured to display, on the display unit, an image containing a target area serving as a target that a subject is caused to gaze at and containing, in a position different from that of the target area, an induction area for inducing a visual hallucination in the subject; an area setting unit configured to set a specific area corresponding to the induction area in the display unit; a determination unit configured to, based on position data on the point of gaze, determine whether the point of gaze is present in the specific area; a computing unit configured to, based on a determination result of the determination unit, calculate point-of-gaze data; and an evaluation unit configured to, based on the point-of-gaze data, calculate evaluation data on the subject.

An evaluation method according to the present disclosure comprising: detecting a position of a point of gaze of a subject on a display unit; displaying, on the display unit, an image containing a target area serving as a target that a subject is caused to gaze at and containing, in a position different from that of the target area, an induction area for inducing a visual hallucination in the subject; setting a specific area corresponding to the induction area in the display unit; based on position data on the point of gaze, determining whether the point of gaze is present in the specific area; based on a determination result, calculating point-of-gaze data; and based on the point-of-gaze data, calculating evaluation data on the subject.

An evaluation program according to the present disclosure for causing a computer to execute processes of detecting a position of a point of gaze of a subject on a display unit; displaying, on the display unit, an image containing a target area serving as a target that a subject is caused to gaze at and containing, in a position different from that of the target area, an induction area for inducing a visual hallucination in the subject; setting a specific area corresponding to the induction area in the display unit; based on position data on the point of gaze, determining whether the point of gaze is present in the specific area; based on a determination result, calculating point-of-gaze data; and based on the point-of-gaze data, calculating evaluation data on the subject.

Advantageous Effects of Invention

According to the disclosure, it is possible to provide an evaluation device, an evaluation method, and an evaluation program that make it possible to make an evaluation on cognitive dysfunction and brain dysfunction accurately.

Brief Description of Drawings

FIG. 1 is a diagram schematically illustrating an example of an evaluation device according to an embodiment.
FIG. 2 is a functional block diagram illustrating an example of the evaluation device.
FIG. 3 is a diagram illustrating and example an evaluation image that is displayed on a display unit.
FIG. 4 is a diagram illustrating another example of the evaluation image that is displayed on the display unit.
FIG. 5 is a flowchart illustrating an example of an evaluation method according to the embodiment.
FIG. 6 is a flowchart illustrating the example of the evaluation method according to the embodiment.

Description of Embodiments

An embodiment of an evaluation device, an evaluation method, and an evaluation program according to the disclosure will be described below based on the drawings. The embodiment does not limit the invention. The components in the embodiment described below include those easily replaceable by those skilled in the art or those substantially the same.

In following description below, description will be given on a positional relationship among components, setting a three-dimensional global coordinate system. A direction parallel to a first axis on a given plane is set for an X-direction, a direction parallel to a second axis on a given plane orthogonal to the first axis is set for a Y-axis, and a direction parallel to a third axis orthogonal to each of the first axis and the second axis is set for a Z-axis direction. The given planes include an XY-plane.

Evaluation Device

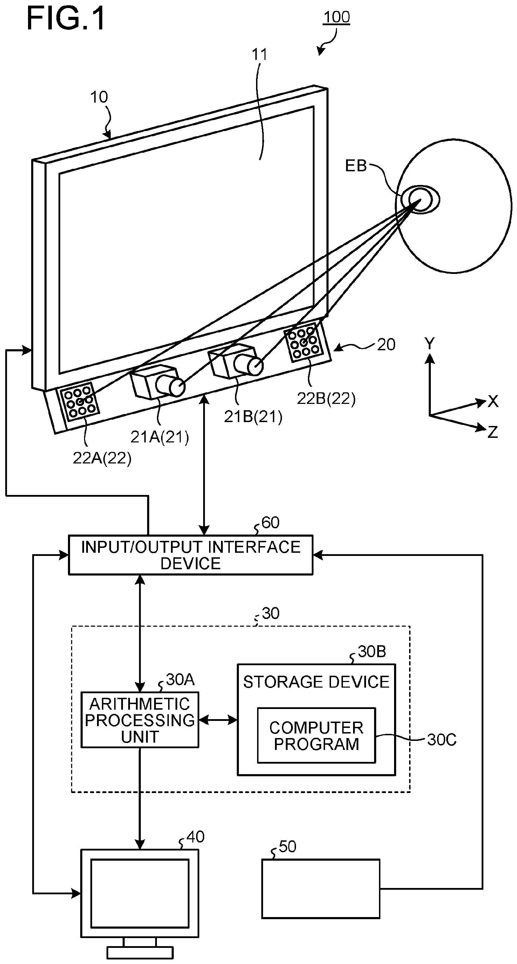

FIG. 1 is a diagram schematically illustrating an example of an evaluation device 100 according to the embodiment. The evaluation device 100 according to the embodiment detects lines of sight of a subject and makes an evaluation on cognitive dysfunction and brain dysfunction using a detection result. As the evaluation device 100, various devices capable of detecting lines of sight of a subject, such as, a device that detects a line of sight based on a position of a pupil of the subject and a position of a corneal reflex image or a device that detects a line of sight based on a position of an inner corner of an eye of the subject and a positon of an iris, are usable.

As illustrated in FIG. 1, the evaluation device 100 includes a display device 10, an image acquisition device 20, a computer system 30, an output device 40, an input device 50, and an input/output interface device 60. The display device 10, the image acquisition device 20, the computer system 30, the output device 40, and the input device 50 perform data communications via the input/output interface device 60. Each of the display device 10 and the image acquisition device 20 includes a drive circuit not illustrated in the drawing.

The display device 10 includes a flat panel display, such as a liquid crystal display (LCD) or an organic electroluminescence display (OLED). In the embodiment, the display device 10 includes a display unit 11. The display unit 11 displays information, such as an image. The display unit 11 is substantially parallel to the XY-plane. The X-axis direction is a left-right direction of the display unit 11, the Y-axis direction is an up-down direction of the display unit 11, and the Z-axis direction is a depth direction orthogonal to the display unit 11. The display device 10 may be a head mounted display. In the case of a head-mounted display, such a configuration as that of the image acquisition device 20 is arranged in a head mounted module.

The image acquisition device 20 acquires image data of left and right eye balls EB of the subject and transmits the acquired image data to the computer system 30. The image acquisition device 20 includes an imaging device 21. The imaging device 21 acquires image data by capturing images of the left and right eyeballs EB of the subject. The imaging device 21 includes various cameras corresponding to a method of detecting a line of sight of the subject. For example, in the case of a system that detects a line of sight based on a position of a pupil of a subject and a position of a corneal reflex image, the imaging device 21 includes an infrared camera and includes an optical system that can transmit near-infrared light of a wavelength of 850 [nm] and an imaging element capable of receiving the near-infrared light. In the case of a system that detects a line of sight based on a position of an inner corner of an eye of a subject and a positon of an iris, the imaging device 21 includes a visible light camera. The imaging device 21 outputs a frame synchronization signal. The period of the frame synchronization signal can be set at, for example, 20 [msec]; however, the period is not limited to this. The imaging device 21 can have a configuration of a stereo camera including a first camera 21A and a second camera 21B; however, the configuration is not limited to this.

Furthermore, for example, in the case of a system that detects a line of sight based on a position of a pupil of a subject and the position of a corneal reflex image, the image acquisition device 20 includes an illuminating device 22 that illuminates the eyeballs EB of the subject. The illuminating device 22 includes an LED (light emitting diode) light source and is capable of emitting near-infrared light of, for example, a wavelength of 850 [nm]. Note that, for example, in the case of a system that detects a line-of-sight vector based on a position of an inner corner of an eye of a subject and a positon of an iris, the illuminating device 22 need not be arranged. The illuminating device 22 emits detection light such that the detection light is synchronized with a frame synchronization signal of the imaging device 21. The illuminating device 22 can be configured to include, for example, a first light source 22A and a second light source 22B; however, the configuration is not limited to this.

The computer system 30 controls operations of the evaluation device 100 overall. The computer system 30 includes an arithmetic processing unit 30A and a storage device 30B. The arithmetic processing unit 30A includes a microprocessor, such as a CPU (central processing unit). The storage device 30B includes a memory or a storage, such as a ROM (read only memory) or a RAM (random access memory). The arithmetic processing unit 30A performs arithmetic processing according to a computer program 30C that is stored in the storage device 30B.

The output device 40 includes a display device, such as a flat panel display. Note that the output device 40 may include a printing device. The input device 50 is operated and thus generates input data. The input device 50 includes a keyboard or a mouse for computer systems. Note that the input device 50 may include a touch sensor that is arranged in the display unit of the output device 40 serving as a display device.

The evaluation device 100 according to the embodiment is a device in which the display device 10 and the computer system 30 are independent from each other. Note that the display device 10 and the computer system 30 may be integrated. For example, the evaluation device 100 may include a tablet personal computer. In this case, a display device, an image acquisition device, a computer system, an input device, an output device, etc., may be installed in the tablet personal computer.

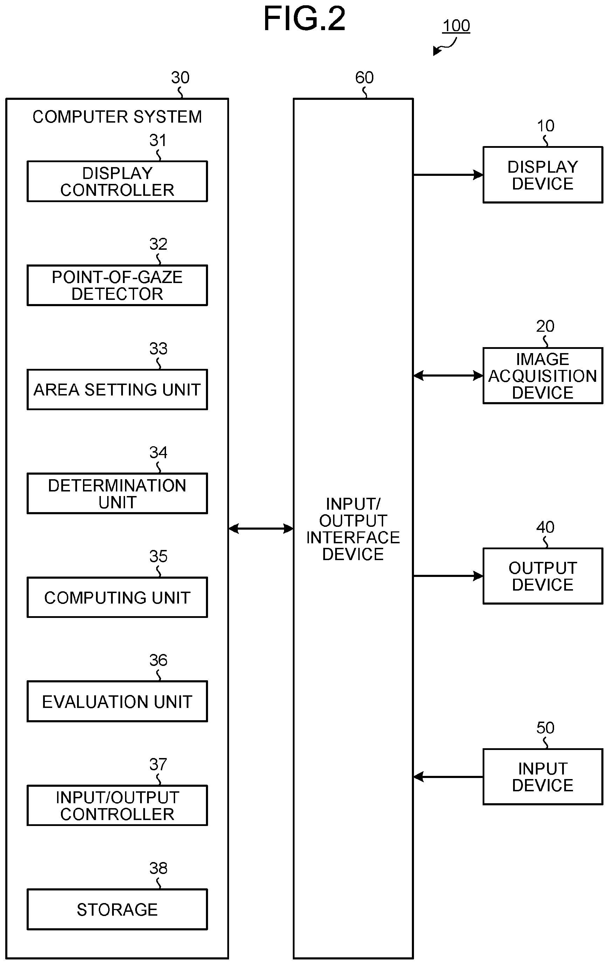

FIG. 2 is a functional block diagram illustrating an example of the evaluation device 100. As illustrated in FIG. 2, the computer system 30 includes a display controller 31, a point-of-gaze detector 32, an area setting unit 33, a determination unit 34, a computing unit 35, an evaluation unit 36, an input/output controller 37, and a storage 38. Functions of the computer system 30 are implemented by the arithmetic processing unit 30A and the storage device 30B (see FIG. 1). Part of the functions of the computer system 30 may be implemented outside the evaluation device 100.

The display controller 31 displays, on the display unit, an evaluation image (image) containing a target area serving as a target that the subject is caused to gaze at and containing, in a position different from that of the target area, an induction area for inducing visual hallucinations in a subject suffering from dementia with Lewy bodies among subjects. In the evaluation image, the value of at least any one of sharpness, luminance, contrast and chroma of the induction area is lower than that of the target area. In the evaluation image, the value of at least any one of sharpness, luminance, contrast and chroma of the target area is higher than that of the surroundings. The evaluation image is a photographic image obtained by capturing an image of a scenery where a subject in the target area is present on a front side in the depth direction and an inducing area and a subject in the inducing area is present on a back side in the depth direction, with the subject in the target area serving as a position of a focal point. A mode of display of the evaluation image may be any one of a still image and a moving image.

The point-of-gaze detector 32 detects position data on a point of gaze of the subject. In the embodiment, based on the image data on the left and right eyeballs EB of the subject that is acquired by the image acquisition device 20, the point-of-gaze detector 32 detects a line-of-sight vector of the subject that is set by the three-dimensional global coordinate system. The point-of-gaze detector 32 detects, as the position data on the point of gaze of the subject, position data on an intersection between the detected line-of-sight vector of the subject and the display unit 11 of the display device 10. In other words, in the embodiment, the position data on the point of gaze is position data on the intersection between the line-of-sight vector of the subject that is set by the three-dimensional global coordinate system and the display unit 11 of the display device 10. In the embodiment, the point of gaze is a specified point on the display unit 11 that is gazed at by the subject and thus is specified. The point-of-gaze detector 32 detects the position data on the point of gaze of the subject at intervals of a sampling period that is set. For the sampling period, it is possible to set, for example, a period (for example, every 20 [msec]) of the frame synchronization signal that is output from the imaging device 21.

In the display unit 11, the area setting unit 33 sets a specific area corresponding to the induction area and a comparison area corresponding to the target area on the display unit 11. In the embodiment, the positions of the induction area and the target area differ from each other. Thus, the area setting unit 33 sets the specific area and the comparison area independently.

During the period in which the area setting unit 33 sets the specific area and the comparison area, the determination unit 34 determines whether the point of gaze is present in the specific area and the comparison area based on the position data on the point of gaze and outputs determination data. The determination unit 34 determines whether the point of gaze is present in the specific area and the comparison area at intervals of the set determination period. The determination period can be, for example, the same period as the period (for example, every 20 [msec]) of the frame synchronization signal that is output from the imaging device 21. In this case, the period of determining by the determination unit 34 is the same as the period of sampling by the point-of-gaze detector 32.

Based on the determination data of the determination unit 34, the computing unit 35 calculates point-of-gaze data presenting the course of shift of the point of gaze during the period in which the specific area and the comparison area described above are set. The computing unit 35 calculates, as the point-of-gaze data, for example, at last any one of reach time data, shifting number-of-times data and presence time data and last area data. The reach time data represents a time to a reach time at which the point of gaze reaches the specific area for the first time. The shifting number-of-times data represents the number of times for which the position of the point of gaze shifts between the specific area and the comparison area until the point of gaze reaches the specific area for the first time. The presence time data represents a presence time during which the point of gaze is present in the specific area during a period of display of the reference images. The last area data represents an area in which the point of gaze is present lastly during the time of display among the specific area and the comparison area. The computing unit 35 includes a timer that detects a time elapsing from display of the evaluation image on the display unit 11 and a counter that counts the number of times for which the determination unit 34 determines that the point of gaze is present in the specific area and the comparison area.

Based on the point-of-gaze data, the evaluation unit 36 calculates evaluation data on the subject. The evaluation data contains data for making an evaluation on whether the subject is being capable of gazing at a specific subject and a comparison subject that are displayed on the display unit 11.

The input/output controller 37 acquires data from at least one of the image acquisition device 20 and the input device 50 (such as the image data on the eyeballs EB and input data). The input/output controller 37 outputs data to at least one of the display device 10 and the output device 40.

The storage 38 stores the determination data, the point-of-gaze data (presence time data) and the evaluation data described above. The storage 38 stores an evaluation program for causing a computer to execute processes of detecting a position of a point of gaze of a subject on the display unit 11; displaying, on the display unit 11, an evaluation image IM1 containing a target area M1 serving as a target that a subject is caused to gaze at and containing, in a position different from that of the target area M1, induction areas C1 and C2 for inducing visual hallucinations in a subject suffering from dementia with Lewy bodies among subjects; setting, in the display unit 11, specific areas A1 and A2 corresponding to the induction areas C1 and C2 and a comparison area B corresponding to the target area M1; based on position data on the point of gaze, determining whether the point of gaze is present in the specific areas A1 and A2 and the comparison area B; based on a determination result, calculating point-of-gaze data; and, based on the point-of-gaze data, calculating evaluation data on the subject.

Evaluation Method

The evaluation method according to the embodiment will be described next. In the evaluation method according to the embodiment, by using the evaluation device 100 described above, an evaluation on cognitive dysfunction and brain dysfunction of the subject is made. In the embodiment, for example, the case where an evaluation is made on dementia with Lewy bodies serving as cognitive dysfunction and brain dysfunction is described.

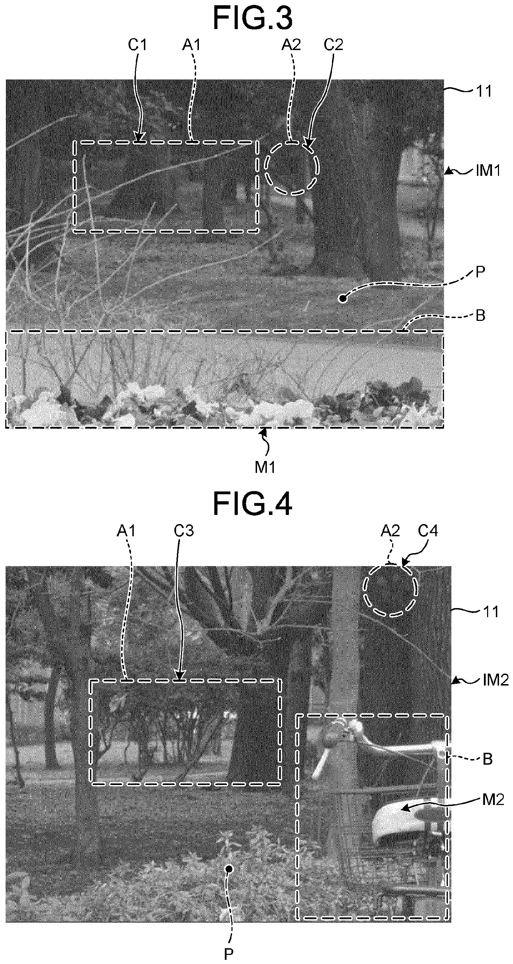

FIG. 3 is a diagram illustrating an example of the evaluation image that is displayed on the display unit 11. As illustrated in FIG. 3, the display controller 31 causes the display unit 11 to display a photographic image as the evaluation image IM1. The evaluation image IM1 is a photographic image on which flowers are on a lower side in the drawing and trees are on an upper side in the drawing. The evaluation image IM1 is a photographic image obtained by capturing an image of a scenery in which the flowers are present on the front side in the depth direction and the trees are present on the back side in the depth direction, with the flowers serving as a position of a focal point. Thus, in the evaluation image IM1, the sharpness, luminance, and contrast of the area with the flowers are higher than those of surrounding areas. In the embodiment, the evaluation image IM1 contains the area with the flowers serving as a subject as the target area M1. The target area M1 is an area serving as a target that the subject is caused to gaze at.

A symptom that visual hallucinations are induced is known as one of symptoms of dementia with Lewy bodies. In the case where a visual hallucination is induced in a subject suffering from dementia with Lewy bodies when the subject gazes at an image, the subject tends to gaze at an area serving as a subject of the visual hallucination. When an evaluation on such dementia with Lewy bodies is made, an image containing an area that induces visual hallucinations is shown to a subject.

In the evaluation image IM1 of the embodiment, the area of the trees on the back side is different from the target area M1 in the following aspects.

1. The focus is on the flowers and the trees are out of focus. In other words, the area of the trees has a lower sharpness than that of the target area M1.
2. The area of the trees has a lower luminance than that of (is darker than) the target area M1.
3. The area of the trees has a lower contrast than that of the target area M1.

In the case where the evaluation image IM1 is a color image, an image in which the area of the trees has a lower chroma than that of the target area M1 is used.

As a result, in the evaluation image IM1, the area of the trees contains areas that tend to induce visual hallucinations in a subject suffering from dementia with Lewy bodies. For example, an area C1 that is part of the area of the trees serves as an area that induces visual hallucinations of the figure of a person, and the like, in a subject suffering from dementia with Lewy bodies. The area C1 is referred to as an induction area C1 below. For example, an area C2 that is part of the area of the trees serves as an area that induces visual hallucinations of the face of a person, and the like, in a subject suffering from dementia with Lewy bodies. The area C2 is referred to as an induction area C2 below.

On the other hand, the induction areas C1 and C2 are areas that are merely part of the area of the trees whose sharpness, luminance and contrast (or chroma) are low to a subject not suffering from dementia with Lewy bodies and are areas that tend not to induce visual hallucinations of the figure of a person, the face of a person, etc. Thus, in the case where the subject is not suffering from dementia with Lewy bodies, when the evaluation image IM1 is displayed on the display unit 11, the subject is highly likely to gaze at the target area M1 whose sharpness, luminance and contrast are high.

In the embodiment, for example, it is possible to use an image with which a significant difference is shown between a subject not suffering from dementia with Lewy bodies and a subject suffering from dementia with Lewy bodies after evaluations are made on multiple subjects. In other words, it is possible to use an image containing a target area that a subject not suffering from dementia with Lewy bodies more tends to gaze at and an induction area that a subject not suffering from dementia with Lewy bodies more tends not to gaze at and a subject suffering from dementia with Lewy bodies more tends to gaze at.

Furthermore, using the above-described photographic image as the evaluation image IM1 more assuredly enables a subject not suffering from dementia with Lewy bodies to gaze at the subject in the target area M1 on the front side. Furthermore, the induction areas C1 and C2 that cause a less sense of discomfort and that is natural to a subject not suffering from dementia with Lewy bodies are contained in the evaluation image IM1. Accordingly, a subject not suffering from dementia with Lewy bodies is inhibited from gazing at the induction areas C1 and C2.

During the period in which the evaluation image IM1 is displayed as described above, the area setting unit 33 sets each of the specific areas A1 and A2 corresponding to the induction areas C1 and C2. The area setting unit 33 sets the comparison area B corresponding to the target area M1. The area setting area 33 is able to set each of the specific areas A1 and A2 in an area containing at least part of the induction areas C1 and C2. The area setting unit 33 is able to set the comparison area B in an area containing at least part of the target area M1. In the embodiment, the area setting unit 33 sets the specific area A1 in a rectangular area containing the induction are C1 and sets a specific area A2 in a circular area containing the induction area C2. The area setting unit 33 sets the comparison area B in a rectangular area containing the target area M1.

During the period in which the evaluation image IM1 is displayed, the point-of-gaze detector 32 detects position data on the point of gaze P of the subject at intervals of the set sampling period (for example, 20 [msec]). When the position data on the point of gaze P of the subject is detected, the determination unit 34 determines whether the point of gaze of the subject is present in the specific areas A1 and A2 and the comparison area B and outputs determination data. Thus, the determination unit 34 outputs determination data at intervals of the same determination period as the above-described sampling period.

Based on the determination data, the computing unit 35 calculates point-of-gaze data representing the course of shift of the point of gaze P during the period of display. The computing unit 35 calculates, as the point-of-gaze data, for example, presence time data, shifting number-of-times data, last area data, and reach time data.

The presence time data represents a presence time during which the point of gaze P is present in the specific areas A1 and A2. In the embodiment, the larger the number of times for which the determination unit 34 determines that the point of gaze is present in the specific areas A1 and A2 is, the more it can be estimated that the presence time in which the point of gaze P is present in the specific areas A1 and A2 is long. Thus, the presence time data can be the number of times for which the determination unit 34 determines that the point of gaze is present in the specific areas A1 and A2 or the comparison area B. In other words, the computing unit 35 can set count values CNTA1, CNTA2 and CNTB of the counter for the presence time data. The count value CNTA1 is the number of times for which it is determined that the point of gaze is present in the specific area A1. The count value CNTA2 is the number of times for which it is determined that the point of gaze is present in the specific area A2. The count value CNTB is the number of times for which it is determined that the point of gaze is present in the comparison area B.

The shifting number-of-times data represents the number of times for which the position of the point of gaze P shifts between the specific areas and the comparison area B until the point of gaze P reaches the specific area A1 or the specific area A2. Thus, the computing unit 35 is able to count how many times the point of gaze P shifts between the specific areas A1 and A2 and the comparison area B and set, for the shifting number-of-times data, the result of counting until the point of gaze P reaches the specific area A1 or A2.

The last area data represents an area in which the point of gaze P is present lastly among the specific areas A1 and A2 and the comparison area B, that is, the area at which the subject gazes lastly as an answer. By updating area in which the point of gaze P is present on every detection of the point of gaze P, the computing unit 35 is able to set, for the last area data, the detection result at the time when displaying the evaluation image IM1 ends.

The reach time data represents the time from the time at which displaying the evaluation image IM1 is started until the time at which the point of gaze P reaches the specific area A1 or A2 for the first time. Thus, by measuring the elapse of time from the start of display with a timer T1 and, at the time when the point of gaze reaches the specific area A1 or the specific area A2 for the first time, detecting a measured value of the timer T1 as a flag value FA1 or a flag value FA2, the computing unit 35 is able to set a detection result of the timer T1 for the reach time data.

The evaluation unit 36 calculates an evaluation value based on the presence time data, the shifting number-of-times data, the last area data, and the reach time data and calculates evaluation data based on the evaluation value. For example, the data value of the last area data is D1, the data values of the presence time data are D2 to D4, the data value of the reach time data is D5, and the data value of the shifting number-of-times data is D6. Note that the data value D1 of the last area data is set at 1 when the last point of gaze P of the subject is present in the specific area A1, is set at 0.8 when the last point of gaze P is present in the specific area A2, and is set at 0 when the last point of gaze P is present in the comparison area B. For each of the data values D2 to D4 of the presence time data, seconds during which the point of gaze P is present in each of the specific areas A1 and A2 and the comparison area B are set. For the data values D2 to D4, an upper limit that is seconds shorter than the period during which the evaluation image IM1 is displayed may be set. For the data value D5 of the reach time data, an inverse of the reach time (for example, $1/(\text{reach time})\div 10$) (10: a coefficient for keeping the minimum value of the reach time at 0.1 second and keeping the reach time evaluation value at 1 or smaller). As for the data value D6 of the shifting number-of-times data, the count value is used directly. Note that an upper limit may be set for the data value D6 as appropriate.

In this case, an evaluation value ANS is represented by, for example, as follows.

$$\text{ANS}=D1\cdot K1+D2\cdot K2+D3\cdot K3-D4\cdot K4+D5\cdot K5-D6\cdot K6$$

K1 to K6 are constants for weighting. The constants K1 to K6 can be set as appropriate. K4=0 may be met. In other words, the data value D4 of the presence time data on the comparison area B need not be contained in the evaluation.

The value of the evaluation value ANS represented by the equation above increases when the data value D1 of the last area data is 1 or 0.8, when the data values D2 and D3 of the presence time data are large, when the data value D4 of the presence time data is small, when the data value D5 of the reach time data is large, or when the data value D6 of the shifting number-of-times data is small. In other words, the evaluation value ANS increases as the last point of gaze P is present in the specific area A1 or A2, the time during which the point of gaze P is present in the specific areas A1 and A2 is long, the time during which the point of gaze P is present in the comparison area B is short, the reach time taken by the point of gaze P to reach the specific area A1 or A2 from the time at which the period of display starts is short, or the number of times for which the point of gaze P shifts between areas is small.

Furthermore, the evaluation value ANS decreases when the data value D1 of the last area data is 0, when the data values D2 and D3 of the presence time data are small, when the data value D4 of the presence time data is large, when the data value D5 of the reach time data is small, or when the value of the data value D6 of the shifting number-of-times data is large. In other words, the evaluation value ANS decreases as the last point of gaze P is present in the comparison area B, the time during which the point of gaze P is present in the specific areas A1 and A2 is short, the time during which the point of gaze P is present in the comparison area B is long, the reach time taken by the point of gaze P to reach the specific area A1 or A2 from the time at which the period of display starts is long or the point of gaze P does not reach the specific area A1 or A2, or the number of times for which the point of gaze P shifts between areas is large.

Accordingly, by determining whether the evaluation value ANS is equal to or larger than a given value, the evaluation unit 36 is able to calculate evaluation data. For example, when the evaluation value ANS is smaller than the given value, it is possible to evaluate that the subject is less likely to be suffering from dementia with Lewy bodies. When the evaluation value ANS is equal to or larger than the given value, it is possible to evaluate that the subject is highly likely to be suffering from dementia with Lewy bodies.

The evaluation unit 36 is able to store the value of the evaluation value ANS in the storage 38. For example, the evaluation values ANS on the same subject may be stored cumulatively and an evaluation may be made in comparison with an evaluation value in the past. For example, when the evaluation value ANS is lower than the evaluation value in the past, it is possible to evaluate that the brain function has improved compared to the previous evaluation. When the cumulative value of evaluation values ANS is gradually decreasing, it is possible to evaluate that the brain function is gradually improving.

The evaluation unit 36 may make an evaluation using the presence time data, the shifting number-of-times data, the last area data, and the reach-time data independently or in combination. For example, when the point of gaze P reaches the comparison area B incidentally during seeing of many objects, the data value D6 of the shifting number-of-times data increases. In this case, it is possible to make an evaluation in combination with the data value D4 of the presence time data described above. For example, in the case where the presence time is long even when the number of times of shifting is small, it is possible to evaluate that the comparison area B can be gazed at. Furthermore, when the number of times of shifting is small and the time of presence is short, it is possible to evaluate that the point of gaze P incidentally passes through the comparison area B.

In the case where the number of times of shifting is small, when the last area is the compassion area B, for example, it is possible to evaluate that the compassion area B is reached in few shifts of the point of gaze. On the other hand, in the aforementioned case where the number of times of shifting is small, when the last area is not the comparison area B, for example, it is possible to evaluate that the point of gaze P incidentally passes though the comparison area B. Thus, it is possible to calculate evaluation data based on the course of shift of the point of gaze by making an evaluation using the point-of-gaze data and thus it is possible to reduce the effect of contingency.

In the embodiment, when the evaluation unit 36 outputs evaluation data, the input/output controller 37 is able to cause the output device 40 to output, for example, character data of "The subject seems to be less likely to be suffering from dementia with Lewy bodies.", character data "The subject seems to be highly likely to be suffering from dementia with Lewy bodies.", or the like. When the evaluation value ANS on the same subject is lower than the evaluation value ANS in the past, the input/output controller 37 is able to cause the output device 40 to output character data of "The brain function has improved", or the like.

FIG. 4 is a diagram illustrating another example of the evaluation image that is displayed on the display unit 11. As illustrated in FIG. 4, the display controller 31 causes the display unit 11 to display a photographic image as an evaluation image IM2. The evaluation image IM2 is a photographic image on which a bicycle is on a lower right side in the drawing and trees are on an upper side in the drawing. The evaluation image IM2 is a photographic image obtained by capturing an image of a scenery in which the bicycle is on the front side in the depth direction and the trees are on the back side in the depth direction, with the bicycle serving as a position of a focal point. Thus, in the evaluation image IM2, the sharpness, luminance, and the contrast of the area with the bicycle are higher than those of surrounding areas. In the embodiment, the evaluation image IM2 contains the area with the bicycle serving as a subject as the target area M2. The target area M2 is an area serving as a target that the subject is caused to gaze at.

On the other hand, in the evaluation image IM2, the area of the trees on the back side is different from the target area M2 in the following aspects.

4. The focus is on the bicycle and the trees are out of focus. In other words, the area of the trees has a lower sharpness than that of the target area M2.

5. The area of the trees has a lower luminance than that of (is darker) the target area M2.

6. The area of the trees has a lower contrast than that of the target area M2.

In the case where the evaluation image IM2 is a color image, an image in which the area of the trees has a lower chroma than that of the target area M2 is used.

As a result, an area that tends to induce visual hallucinations in a subject suffering from dementia with Lewy bodies is present in the evaluation image IM2. For example, an area C3 that is part of the area of the trees serves as an area that induces a visual hallucination of the figure of a person, or the like, in a subject suffering from dementia with Lewy bodies. The area C3 is referred to as an induction area C3 below. An area C4 that is part of the area of the trees serves as an area that induces a visual hallucination of the face of a person in a subject suffering from dementia with Lewy bodies. The area C4 is referred to as an induction area C4 below.

During the period in which the evaluation image IM2 is displayed, the area setting unit 33 sets each of the specific areas A1 and A2 corresponding to the induction areas C3 and C4. The area setting unit 33 sets a comparison area B corresponding to the target area M2. The area setting unit 33 is able to set the specific areas A1 and A2 in areas containing at least part of the induction areas C3 and C4, respectively. The area setting unit 33 is able to set the comparison area B in an area containing at least part of the target area M2. In the embodiment, the area setting unit 33 sets the specific area A1 in a rectangular area containing the induction area C3 and sets the specific area A2 in a circular area containing the induction area C4. The area setting unit 33 sets the comparison area B in a rectangular area containing the target area M2.

The display controller 31 is able to display the evaluation image IM2 illustrated in FIG. 4 on the display unit 11 for a given time after displaying the evaluation image IM1 illustrated in FIG. 3 for a given time. As described above, displaying multiple types of the evaluation images IM1 and IM2 on the display unit 11 enables accurate evaluation on the subject.

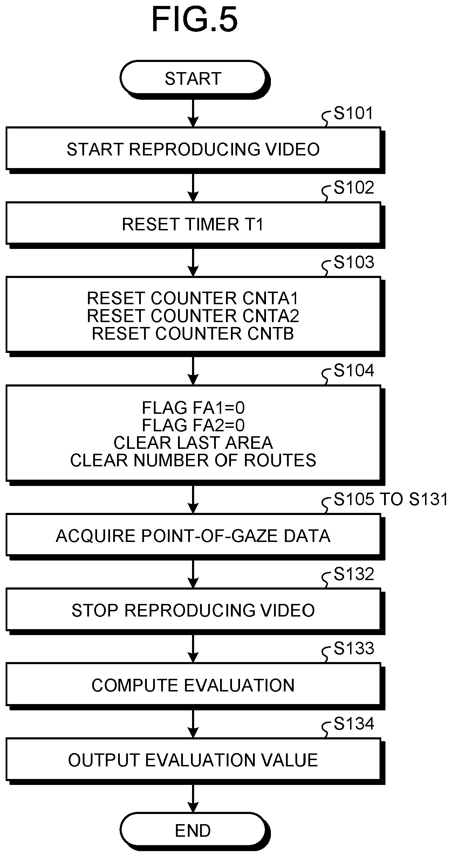

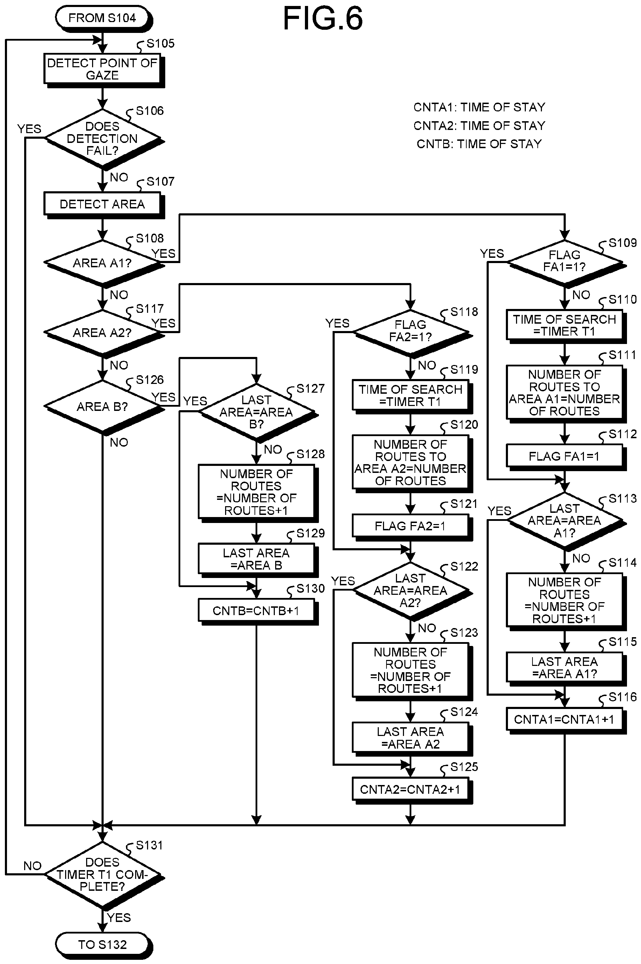

An example of the evaluation method according to the embodiment will be described with reference to FIGS. 5 and 6. FIGS. 5 and 6 are flowcharts representing the example of the evaluation method according to the embodiment. In the following example, description will be given exemplifying the case where the evaluation image IM1 is reproduced as a video (evaluation video). As illustrated in FIG. 5, the display controller 31 starts reproduction of an evaluation image (display of the evaluation image IM1) (step S101). The evaluation image IM1 may be displayed in the interval between displays of images for making other evaluations on a subject. After reproduction of the evaluation image IM1 is started, the timer T1 is reset (step S102) and count values CNTA1, CNTA2 and CNTB are reset (step S103). Furthermore, the display controller 31 sets the flag values FA1 and FA2 at 0 and clears the last area and the number of routes (step S104).

As illustrated in FIG. 6, the point-of-gaze detector 32 detects position data on the point of gaze P of the subject on the display unit 11 at intervals of a sampling period that is set (for example, 20 [msec]) with the video being displayed on the display unit 11 and shown to the subject (step S105). When position data is detected (NO at step S106), the determination unit 34 determines an area in which the point of gaze P is present based on the position data (step S107). When the position data is not detected (YES at step S106), the process at and after step S131 is performed.

When it is determined that the point of gaze P is in the specific area A1 (YES at step S108), the computing unit 35 determines whether the flag value FA1 is 1, that is, whether the point of gaze P reaches the specific area A1 for the first time (1: reached, 0: not reached) (step S109). When the flag value FA1 is 1 (YES at step S109), the computing unit 35 skips steps S110 to S112 below and performs the process of step S113 to be described below.

When the flag value FA1 is not 1, that is, when the point of gaze P reaches the specific area A1 for the first time (NO at step S109), the computing unit 35 extracts a measurement result of the timer T1 as reach time data (step S110) and saves a cumulative number representing how many times the point of gaze P shifts among areas until the point of gaze P reaches the specific area A1 (step S111). Thereafter, the computing unit 35 changes the flag value to 1 (step S112).

The computing unit 35 determines whether the area in which the point of gaze P is present in the last detection, that is, the last area is the specific area A1 (step S113). When it is determined that the last area is the specific area A1 (YES at step S113), the computing unit 35 skips step S114 and step S115 below and performs the process of step S116 to be described below. When it is determined that the last area is not the specific area A1 (NO at step S113), the computing unit 35 incrementally increases by 1 the cumulative number representing how many times the point of gaze P shifts among areas (step S114), sets the specific area A1 for the last area (step S115), and incrementally increases by 1 the count value CNTA1 representing the data on the time of presence in the specific area A1 (step S116). Thereafter, the computing unit 35 performs the process at and after step S131 to be described below.

When it is determined that the point of gaze P is not present in the specific area A1 (NO at step S108), the computing unit 35 determines whether the point of gaze P is present in the specific area A2 (step S117). When it is determined that the point of gaze P is present in the specific area A2 (YES at step S117), the computing unit 35 determines whether the flag value FA2 is 1, that is, whether the point of gaze P reaches the specific area A2 for the first time (1: reached, 0: not reached) (step S118). When the flag value FA2 is 1 (YES at step S118), the computing unit 35 skips steps S119 to S121 below and performs the process of step S122 to be described below.

When the flag value FA2 is not 1, that is, when the point of gaze P reaches the specific area A2 for the first time (NO at step S118), the computing unit 35 extracts a measurement result of the timer T1 as reach time data (step S119) and saves a cumulative number representing how many times the point of gaze P shifts among areas until the point of gaze P reaches the specific area A2 (step S120). Thereafter, the computing unit 35 changes the flag value to 1 (step S121).

The computing unit 35 determines whether the area in which the point of gaze P is present in the last detection, that is, the last area is the specific area A2 (step S122). When it is determined that the last area is the specific area A2 (YES at step S122), the computing unit 35 skips steps S123 and step S124 below and performs the process of step S125 to be described below. When it is determined that the last area is not the specific area A2 (NO at step S122), the computing unit 35 incrementally increases by 1 the cumulative number representing how many times the point of gaze P shifts among areas (step S123), sets the specific area A2 for the last area (step S124), and incrementally increases by 1 the count value CNTA2 representing the data on the time of presence in the specific area A2 (step S125). Thereafter, the computing unit 35 performs the process at and after step S131 to be described below.

When it is determined that the point of gaze P is not present in the specific area A2 (NO at step S117), the computing unit 35 determines whether the point of gaze P is present in the comparison area B (step S126). When it is determined that the point of gaze P is present in the comparison area B (YES at step S126), the computing unit 35 determines whether the area in which the point of gaze P is present in the last detection, that is, the last area is the comparison area B (step S127). When it is determined that the last area is the comparison area B (YES at step S127), the computing unit 35 skips step S128 and step S129 below and performs the process of step S130. When it is determined that the last area is not the comparison area B (NO at step S127), the computing unit 35 incrementally increases by 1 the cumulative number representing how many times the point of gaze P shifts among areas (step S128), sets the comparison area B for the last area (step S129), and incrementally increases by 1 the count value CNTB representing the data on the time of presence in the comparison area B (step S130). After step S130, when it is determined that the point of gaze P is not present in the comparison area B at step S126 (NO at step S126), the computing unit 35 performs the process at and after step S131 to be described below.

Thereafter, based on a detection result of the detection timer T1, the computing unit 35 determines whether the time at which reproduction of the evaluation image completes is reached (step S131). When it is determined that the time at which reproduction of the evaluation image completes is not reached (NO at step S131), the computing unit 35 performs the process at and after step S105 repeatedly.

When the computing unit 35 determines that the time at which reproduction of the evaluation image completes is reached (YES at step S131), as illustrated in FIG. 5, the display controller 31 stops reproduction of the video relating to an instruction display operation (step S132). After reproduction of the video is stopped, the evaluation unit 36 calculates an evaluation value ANS based on presence time data, shifting number-of-times data, reach time data, and last area data from the result of the above-described process (step S133) and calculates evaluation data based on the evaluation value ANS. Thereafter, the input/output controller 37 outputs the evaluation data that is calculated by the evaluation unit 36 (step S134).

The evaluation device 100 according to the embodiment includes the display unit 11; the point-of-gaze detector 32 that detects a position of a point of gaze of a subject on the display unit 11; the display controller 31 that displays, on the display unit 11, the evaluation image IM1 containing the target area M1 serving as a target that the subject is caused to gaze at and containing, in a positon different from that of the target area M1, the induction areas C1 and C2 for inducing a hallucination in a subject; the area setting unit 33 that sets, in the display unit 11, the specific areas A1 and A2 corresponding to the induction areas C1 and C2; the determination unit 34 that, based on position data on the point of gaze, determines whether the point of gaze is present in the specific area A1 or A2; the computing unit 35 that calculates point-of-gaze data based on a determination result of the determination unit 34, and the evaluation unit 36 that calculates evaluation data on the subject based on the point-of-gaze data.

The evaluation method according to the embodiment includes detecting a position of a point of gaze of a subject on the display unit 11; displaying, on the display unit 11, the evaluation image IM1 containing the target area M1 serving as a target that a subject is caused to gaze at and containing, in a position different from that of the target area M1, the induction areas C1 and C2 for inducing visual hallucinations in a subject; setting, in the display unit 11, the specific areas A1 and A2 corresponding to the induction areas C1 and C2; based on position data on the point of gaze, determining whether the point of gaze is present in the specific area A1 or A2; based on a determination result, calculating point-of-gaze data; and, based on the point-of-gaze data, calculating evaluation data on the subject.

The evaluation program according to the embodiment causes a computer to execute processes of detecting a position of a point of gaze of a subject on the display unit 11; displaying, on the display unit 11, the evaluation image IM1 containing the target area M1 serving as a target that a subject is caused to gaze at and containing, in a position different from that of the target area M1, the induction areas C1 and C2 for inducing visual hallucinations in a subject; setting, in the display unit 11, the specific areas A1 and A2 corresponding to the induction areas C1 and C2; based on position data on the point of gaze, determining whether the point of gaze is present in the specific area A1 or A2; based on a determination result, calculating point-of-gaze data; and, based on the point-of-gaze data, calculating evaluation data on the subject.

In the case where the subject is not suffering from dementia with Lewy bodies, when the evaluation image IM1 is displayed on the display unit 11, the subject is highly likely to gaze at the target area M1 whose sharpness, luminance, and contrast are high. In the case where the subject is suffering from dementia with Lewy bodies, when the evaluation image IM1 is displayed on the display unit 11, the subject is highly likely to gaze at the induction area C1 or C2 that induces visual hallucinations. According to the above-described configuration, because a subject is caused to gaze at the evaluation image IM1 in which the area that tends to induce visual hallucinations in a subject suffering from dementia with Lewy bodies is present, it is possible to make an evaluation not depending on the subjective view of the subject. It is also possible to reduce the effect of contingency because it is possible to calculate evaluation data based on the point-of-gaze data on the subject.

In the evaluation device 100 according to the embodiment, the area setting unit 33 further sets the comparison area B corresponding to the target area M1 in the display unit 11 and the determination unit 34 determines whether the point of gaze is present in the specific areas A1 and A2 and the comparison area B. Accordingly, it is possible to evaluate the subject more accurately.

In the evaluation device 100 according to the embodiment, the values of at least one of sharpness, luminance, contrast and chroma of the induction areas C1 and C2 are lower than that of the target area M1. Accordingly, the area that tends to cause visual hallucinations in a subject suffering from dementia with Lewy bodies can be contained efficiently.

In the evaluation device 100 according to the embodiment, the value of at least one of sharpness, luminance, contrast and chroma of the target area M1 is higher than that of the surroundings. Accordingly, a subject not suffering from dementia with Lewy bodies is to gaze at the target area M1 and thus it is possible to make an accurate evaluation.

In the evaluation device 100 according to the embodiment, the evaluation image IM1 is a photographic image in which the subject in the target area M1 is present on the front side in the depth direction and the subject in the induction areas C1 and C2 is present on the back side in the depth direction and that is captured with the subject in the target area M1 serving as a position of a focal point. This more assuredly enables a subject not suffering from dementia with Lewy bodies to gaze at the subject in the target area M1 on the front side. Furthermore, the induction areas C1 and C2 that are less artificial and natural can be contained in the evaluation image IM1. Thus, it is possible to inhibit a subject not suffering from dementia with Lewy bodies from gazing at the induction areas C1 and C2 and make an accurate evaluation.

In the evaluation device 100 according to the embodiment, the point-of-gaze data contains at least one of reach time data representing a time to a time at which the point of gaze reaches the specific area A1 or A2 for the first time, shifting number-of-times data representing the number of times for which the position of the point of gaze shifts between the specific area A1 or A2 and the multiple comparison areas B until the point of gaze reaches the specific area A1 or A2 for the first time, and presence time data representing presence time of presence in the specific areas A1 and A2 and last area data representing the area in which the point of gaze is present lastly during the time of display among the specific areas A1 and A2 and the comparison area B and the evaluation unit 36 weights the reach time data, shifting number-of-times data, the presence time data and the last area data differently and calculates evaluation data. Thus, it is possible to reduce the effect of contingency more assuredly.

In the evaluation device 100 according to the embodiment, images with which a significant difference is shown between a subject not suffering from dementia with Lewy bodies and a subject suffering from dementia with Lewy bodies as a result of evaluation by the evaluation unit 36 are used as the evaluation images IM1 and IM2. Accordingly, it is possible to accurately make an evaluation on the possibility that a subject would suffer from dementia with Lewy bodies.

The technical scope of the disclosure is not limited to the embodiment described above and changes can be added as appropriate without departing from the purpose of the disclosure. For example, the above-described embodiment has been described, exemplifying the case where the induction areas C1 to C4 that induce visual hallucinations of the figure of a person or the face of a person are contained in the evaluation images IM1 and IM2; however, embodiments are not limited thereto. For example, an induction area that induces a visual hallucination of an animal other than a person, such as a small animal or an insect, may be contained in the evaluation image.

In the above-described embodiment, the example where an evaluation value is calculated using point-of-gaze data on the specific areas A1 and A2 and the comparison area B; however, embodiments are not limited thereto. For example, an evaluation value may be calculated based on only the point-of-gaze data on the specific areas A1 and A2 without using point-of-gaze data on the comparison area B. In this case, for example, the area setting unit 33 is able to set only the specific areas A1 and A2 without setting the comparison area B.

In the above-described embodiment, the case where a photographic image is displayed as an evaluation image has been described; however, the evaluation image is not limited to a photographic image and a drawn or created image may be used.

Industrial Applicability

The evaluation device, the evaluation method and the evaluation program are usable for, for example, a line-of-sight detection device.

Reference Signs List

A1, A2 SPECIFIC AREA, B COMPARISON AREA, C1 TO C4 INDUCTION AREA, IM1, IM2 EVALUATION IMAGE, M1, M2 TARGET AREA, P POINT OF GAZE, T1 TIMER, 10 DISPLAY DEVICE, 11 DISPLAY UNIT, 20 IMAGE ACQUISITION DEVICE, 21 IMAGING DEVICE, 21A FIRST CAMERA, 21B SECOND CAMERA, 22 ILLUMINATING DEVICE, 22A FIRST LIGHT SOURCE, 22B SECOND LIGHT SOURCE, 30 COMPUTER SYSTEM, 30A ARITHMETIC PROCESSING UNIT, 30B STORAGE DEVICE, 30C COMPUTER PROGRAM, 31 DISPLAY CONTROLLER, 32 POINT-OF-GAZE DETECTOR, 33 AREA SETTING UNIT, 34 DETERMINATION UNIT, 35 COMPUTING UNIT, 36 EVALUATION UNIT, 37 INPUT/OUTPUT CONTROLLER, 38 STORAGE, 40 OUTPUT DEVICE, 50 INPUT DEVICE, 60 INPUT/OUTPUT INTERFACE DEVICE, 100 EVALUATION DEVICE

**Claims**

1. An evaluation device (100) comprising:

   a display unit (11);
   a point-of-gaze detector (32) configured to detect a position of a point of gaze of a subject on the display unit (11);
   a display controller (31) configured to display, on the display unit (11), an image containing a target area serving as a target that a subject is caused to gaze at and containing, in a position different from that of the target area, an induction area for inducing a visual hallucination in the subject;
   an area setting unit (33) configured to set a specific area corresponding to the induction area in the display unit (11);
   a determination unit (34) configured to, based on position data on the point of gaze, determine whether the point of gaze is present in the specific area;
   a computing unit (34) configured to, based on a determination result of the determination unit (34), calculate point-of-gaze data; and
   an evaluation unit (36) configured to, based on the point-of-gaze data, calculate evaluation data on the subject, wherein a value of at least one of sharpness, luminance, contrast and chroma of the induction area is lower than that of the target area.

2. The evaluation device (100) according to claim 1, wherein the area setting unit (33) is configured to further set a comparison area corresponding to the target area in the display unit (11), and
   the determination unit (34) is configured to determine whether the point of gaze is present in the specific area and the comparison area.

3. The evaluation device (100) according to claim 1 or 2, wherein a value of at least one of sharpness, luminance, contrast and chroma of the target area is higher than that of surroundings.

4. The evaluation device (100) according to any one of claims 1 to 3, wherein the image is a photographic image in which a subject in the target area is present on a front side in a depth direction and a subject in the induction area is present on a back side in the depth direction and that is captured with the subject in the target area serving as a position of a focal point or an image that is drawn and produced.

5. The evaluation device (100) according to any one of claims 1 to 4, wherein the point-of-gaze data contains at least one of reach time data representing a time to a time at which the point of gaze reaches the specific area for the first time, shifting number-of-times data representing a number of times for which the position of the point of gaze shifts between the specific area and the comparison area until the point of gaze reaches the specific area for the first time, and presence time data representing a presence time during which the point of gaze is present in the specific area and the comparison area and last area data representing an area in which the point of gaze is present lastly during the time of display among the specific area and the comparison area, and
   the evaluation unit (36) is configured to weight the reach time data, the shifting number-of-times data, the presence time data and the last area data and calculates the evaluation data.

6. The evaluation device (100) according to any one of claims 1 to 4, wherein an image with which a significant difference is shown between a subject not suffering from dementia with Lewy bodies and a subject suffering from dementia with Lewy bodies as a result of evaluation by the evaluation unit (36) is used as the image.

7. A computer-implemented evaluation method comprising:

detecting a position of a point of gaze of a subject on a display unit (11) as disclosed in claim 1;
displaying, on the display unit (11), an image containing a target area serving as a target that a subject is caused to gaze at and containing, in a position different from that of the target area, an induction area for inducing a visual hallucination in the subject;
setting a specific area corresponding to the induction area in the display unit (11);
based on position data on the point of gaze, determining whether the point of gaze is present in the specific area;
based on a determination result, calculating point-of-gaze data; and
based on the point-of-gaze data, calculating evaluation data on the subject,
wherein a value of at least one of sharpness, luminance, contrast and chroma of the induction area is lower than that of the target area.

8. An evaluation program for causing a computer to execute processes of

detecting a position of a point of gaze of a subject on a display unit (11) as disclosed in claim 1;
displaying, on the display unit (11), an image containing a target area serving as a target that a subject is caused to gaze at and containing, in a position different from that of the target area, an induction area for inducing a visual hallucination in the subject;
setting a specific area corresponding to the induction area in the display unit (11);
based on position data on the point of gaze, determining whether the point of gaze is present in the specific area;
based on a determination result, calculating point-of-gaze data; and
based on the point-of-gaze data, calculating evaluation data on the subject,
wherein a value of at least one of sharpness, luminance, contrast and chroma of the induction area is lower than that of the target area.

**Patentansprüche**

1. Auswertungsvorrichtung (100), umfassend:

eine Anzeigeeinheit (11);
einen Blickpunktdetektor (32), der konfiguriert ist, um eine Position eines Blickpunkts eines Subjekts auf der Anzeigeeinheit (11) zu detektieren;
eine Anzeigesteuerung (31), die konfiguriert ist, um auf der Anzeigeeinheit (11) ein Bild anzuzeigen, das einen Zielbereich enthält, der als ein Ziel dient, auf das ein Subjekt zu blicken veranlasst wird, und enthaltend, in einer Position, die sich von der des Zielbereichs unterscheidet, einen Induktionsbereich zum Induzieren einer visuellen Halluzination in dem Subjekt;
eine Bereichseinstelleinheit (33), die konfiguriert ist, um einen spezifischen Bereich einzustellen, der dem Induktionsbereich in der Anzeigeeinheit (11) entspricht;
eine Bestimmungseinheit (34), die konfiguriert ist, um basierend auf Positionsdaten über den Blickpunkt zu bestimmen, ob der Blickpunkt im spezifischen Bereich präsent ist;
eine Recheneinheit (34), die konfiguriert ist, um basierend auf einem Bestimmungsergebnis der Bestimmungseinheit (34) Blickpunktdaten zu berechnen; und
eine Auswertungseinheit (36), die konfiguriert ist, um basierend auf den Blickpunktdaten Auswertungsdaten über das Subjekt zu berechnen,
wobei ein Wert von mindestens einem von Schärfe, Luminanz, Kontrast und Chroma des Induktionsbereichs niedriger als der des Zielbereichs ist.

2. Auswertungsvorrichtung (100) nach Anspruch 1, wobei die Bereichseinstelleinheit (33) konfiguriert ist, um ferner einen Vergleichsbereich entsprechend dem Zielbereich in der Anzeigeeinheit (11) einzustellen, und die Bestimmungseinheit (34) konfiguriert ist, um zu bestimmen, ob der Blickpunkt im spezifischen Bereich und dem Vergleichsbereich präsent ist.

3. Auswertungsvorrichtung (100) nach Anspruch 1 oder 2, wobei ein Wert von mindestens einem von Schärfe, Luminanz, Kontrast und Chroma des Zielbereichs höher als der einer Umgebung ist.

4. Auswertungsvorrichtung (100) nach einem der Ansprüche 1 bis 3, wobei das Bild ein fotografisches Bild ist, in dem ein Subjekt im Zielbereich auf einer Vorderseite in einer Tiefenrichtung präsent ist und ein Subjekt im Induktionsbereich auf einer Rückseite in der Tiefenrichtung präsent ist und das mit dem Subjekt im Zielbereich aufgenommen wird, das als eine Position eines Brennpunkts dient, oder ein Bild, das gezeichnet und produziert ist.

5. Auswertungsvorrichtung (100) nach einem der Ansprüche 1 bis 4, wobei die Blickpunktdaten mindestens eines enthalten von Erreichzeitdaten, die eine Zeit bis zu einer Zeit darstellen, zu der der Blickpunkt den spezifischen Bereich zum ersten Mal erreicht, Verschiebungsanzahldaten, die eine Anzahl von Malen darstellen, für die sich die Position des Blickpunkts zwischen dem spezifischen Bereich und dem Vergleichsbereich verschiebt, bis der Blickpunkt den spezifischen Bereich zum ersten Mal erreicht, und Präsenzzeitdaten, die eine Präsenzzeit darstellen, während der der Blickpunkt im spezifischen Bereich und dem Vergleichsbereich präsent ist, und letzten Bereichsdaten, die einen Bereich darstellen, in dem der Blickpunkt zuletzt während der Anzeigezeit zwischen dem spezifischen Bereich und dem Vergleichsbereich präsent ist, und
die Auswertungseinheit (36) konfiguriert ist, um die Erreichzeitdaten, die Verschiebungsanzahldaten, die Präsenzzeitdaten und die letzten Bereichsdaten zu gewichten und die Auswertungsdaten berechnet.

6. Auswertungsvorrichtung (100) nach einem der Ansprüche 1 bis 4, wobei ein Bild mit dem ein signifikanter Unterschied gezeigt wird zwischen einem Subjekt, das nicht an Demenz mit Lewy-Körpern leidet, und einem Subjekt, das als Ergebnis der Auswertung durch die Auswertungseinheit (36) an Demenz mit Lewy-Körpern leidet, als das Bild verwendet wird.

7. Computerimplementiertes Auswertungsverfahren, umfassend:

   Detektieren einer Position eines Blickpunkts eines Subjekts auf einer Anzeigeeinheit (11) wie in Anspruch 1 offenbart;
   Anzeigen, auf der Anzeigeeinheit (11), eines Bilds, das einen Zielbereich enthält, der als ein Ziel dient, auf das ein Subjekt zu blicken veranlasst wird, und enthaltend, in einer Position, die sich von der des Zielbereichs unterscheidet, einen Induktionsbereich zum Induzieren einer visuellen Halluzination in dem Subjekt;
   Einstellen eines spezifischen Bereichs, der dem Induktionsbereich in der Anzeigeeinheit (11) entspricht;
   basierend auf Positionsdaten über den Blickpunkt, Bestimmen, ob der Blickpunkt im spezifischen Bereich präsent ist;
   basierend auf einem Bestimmungsergebnis, Berechnen von Blickpunktdaten; und
   basierend auf den Blickpunktdaten, Berechnen von Auswertungsdaten über das Subjekt,
   wobei ein Wert von mindestens einem von Schärfe, Luminanz, Kontrast und Chroma des Induktionsbereichs niedriger als der des Zielbereichs ist.

8. Auswertungsprogramm, um einen Computer dazu zu veranlassen Prozesse auszuführen zum

   Detektieren einer Position eines Blickpunkts eines Subjekts auf einer Anzeigeeinheit (11) wie in Anspruch 1 offenbart;
   Anzeigen, auf der Anzeigeeinheit (11), eines Bilds, das einen Zielbereich enthält, der als ein Ziel dient, auf das ein Subjekt zu blicken veranlasst wird, und enthaltend, in einer Position, die sich von der des Zielbereichs unterscheidet, einen Induktionsbereich zum Induzieren einer visuellen Halluzination in dem Subjekt;
   Einstellen eines spezifischen Bereichs, der dem Induktionsbereich in der Anzeigeeinheit (11) entspricht;
   basierend auf Positionsdaten über den Blickpunkt, Bestimmen, ob der Blickpunkt im spezifischen Bereich präsent ist;
   basierend auf einem Bestimmungsergebnis, Berechnen von Blickpunktdaten; und
   basierend auf den Blickpunktdaten, Berechnen von Auswertungsdaten über das Subjekt,
   wobei ein Wert von mindestens einem von Schärfe, Luminanz, Kontrast und Chroma des Induktionsbereichs niedriger als der des Zielbereichs ist.

**Revendications**

1. Dispositif d'évaluation (100) comprenant :

   une unité d'affichage (11) ;
   un détecteur de point de regard (32) configuré pour détecter une position d'un point de regard d'un sujet sur l'unité d'affichage (11) ;

un dispositif de commande d'affichage (31) configuré pour afficher, sur l'unité d'affichage (11), une image contenant une zone cible servant de cible sur laquelle un sujet est amené à regarder et contenant, dans une position différente de celle de la zone cible, une zone d'induction pour induire une hallucination visuelle chez le sujet ;

une unité de réglage de zone (33) configurée pour régler une zone spécifique correspondant à la zone d'induction dans l'unité d'affichage (11) ;

une unité de détermination (34) configurée pour, sur la base de données de position sur le point de regard, déterminer si le point de regard est présent dans la zone spécifique ;

une unité de calcul (34) configurée pour, sur la base d'un résultat de détermination de l'unité de détermination (34), calculer des données de point de regard ; et

une unité d'évaluation (36) configurée pour, sur la base des données de point de regard, calculer des données d'évaluation sur le sujet,

dans lequel une valeur d'au moins l'un parmi la netteté, la luminance, le contraste et la chrominance de la zone d'induction est inférieure à celle de la zone cible.

**2.** Dispositif d'évaluation (100) selon la revendication 1, dans lequel l'unité de réglage de zone (33) est configurée pour régler en outre une zone de comparaison correspondant à la zone cible dans l'unité d'affichage (11), et l'unité de détermination (34) est configurée pour déterminer si le point de regard est présent dans la zone spécifique et la zone de comparaison.

**3.** Dispositif d'évaluation (100) selon la revendication 1 ou 2, dans lequel une valeur d'au moins l'un parmi la netteté, la luminance, le contraste et la chrominance de la zone cible est supérieure à celle d'un environnement.

**4.** Dispositif d'évaluation (100) selon l'une quelconque des revendications 1 à 3, dans lequel l'image est une image photographique dans laquelle un sujet dans la zone cible est présent sur un côté avant dans une direction de profondeur et un sujet dans la zone d'induction est présent sur un côté arrière dans la direction de profondeur et qui est capturée avec le sujet dans la zone cible servant de position d'un point focal ou une image qui est dessinée et produite.

**5.** Dispositif d'évaluation (100) selon l'une quelconque des revendications 1 à 4, dans lequel les données de point de regard contiennent au moins l'une parmi des données de temps d'atteinte représentant un temps jusqu'à un temps auquel le point de regard atteint la zone spécifique pour la première fois, des données de nombre de fois de décalage représentant un nombre de fois pendant lesquels la position du point de regard se décale entre la zone spécifique et la zone de comparaison jusqu'à ce que le point de regard atteigne la zone spécifique pour la première fois, et des données de temps de présence représentant un temps de présence pendant lequel le point de regard est présent dans la zone spécifique et la zone de comparaison et des données de dernière zone représentant une zone dans laquelle le point de regard est présent en dernier pendant le temps d'affichage entre la zone spécifique et la zone de comparaison, et l'unité d'évaluation (36) est configurée pour pondérer les données de temps d'atteinte, les données de nombre de fois de décalage, les données de temps de présence et les données de dernière zone et calcule les données d'évaluation.

**6.** Dispositif d'évaluation (100) selon l'une quelconque des revendications 1 à 4, dans lequel une image avec laquelle une différence significative est montrée entre un sujet ne souffrant pas de démence avec des corps de Lewy et un sujet souffrant de démence avec des corps de Lewy à la suite de l'évaluation par l'unité d'évaluation (36) est utilisée en tant qu'image.

**7.** Procédé d'évaluation mis en œuvre par ordinateur comprenant :

détecter une position d'un point de regard d'un sujet sur une unité d'affichage (11) selon la revendication 1 ;

afficher, sur l'unité d'affichage (11), une image contenant une zone cible servant de cible sur laquelle un sujet est amené à regarder et contenant, dans une position différente de celle de la zone cible, une zone d'induction pour induire une hallucination visuelle chez le sujet ;

régler une zone spécifique correspondant à la zone d'induction dans l'unité d'affichage (11) ;

sur la base de données de position sur le point de regard, déterminer si le point de regard est présent dans la zone spécifique ;

sur la base d'un résultat de détermination, calculer des données de point de regard ; et

EP 3 970 624 B1

sur la base des données de point de regard, calculer des données d'évaluation sur le sujet,
dans lequel une valeur d'au moins l'un parmi la netteté, la luminance, le contraste et la chrominance de la zone d'induction est inférieure à celle de la zone cible.

8. Programme d'évaluation pour amener un ordinateur à exécuter des procédés pour

détecter une position d'un point de regard d'un sujet sur une unité d'affichage (11) selon la revendication 1 ;
afficher, sur l'unité d'affichage (11), une image contenant une zone cible servant de cible sur laquelle un sujet est amené à regarder et contenant, dans une position différente de celle de la zone cible, une zone d'induction pour induire une hallucination visuelle chez le sujet ;
régler une zone spécifique correspondant à la zone d'induction dans l'unité d'affichage (11) ;
sur la base de données de position sur le point de regard, déterminer si le point de regard est présent dans la zone spécifique ;
sur la base d'un résultat de détermination, calculer des données de point de regard ; et
sur la base des données de point de regard, calculer des données d'évaluation sur le sujet,
dans lequel une valeur d'au moins l'un parmi la netteté, la luminance, le contraste et la chrominance de la zone d'induction est inférieure à celle de la zone cible.

# FIG.1

# FIG.2

100

30

## COMPUTER SYSTEM

### 31
DISPLAY CONTROLLER

### 32
POINT-OF-GAZE DETECTOR

### 33
AREA SETTING UNIT

### 34
DETERMINATION UNIT

### 35
COMPUTING UNIT

### 36
EVALUATION UNIT

### 37
INPUT/OUTPUT CONTROLLER

### 38
STORAGE

60

INPUT/ OUTPUT INTERFACE DEVICE

10
DISPLAY DEVICE

20
IMAGE ACQUISITION DEVICE

40
OUTPUT DEVICE

50
INPUT DEVICE

# FIG.3

C1    A1         A2   C2

11

IM1

P

B

M1

# FIG.4

A1    C3              A2   C4

11

IM2

B

M2

P

# FIG.5

START

S101
START REPRODUCING VIDEO

S102
RESET TIMER T1

S103
RESET COUNTER CNTA1
RESET COUNTER CNTA2
RESET COUNTER CNTB

S104
FLAG FA1=0
FLAG FA2=0
CLEAR LAST AREA
CLEAR NUMBER OF ROUTES

S105 TO S131
ACQUIRE POINT-OF-GAZE DATA

S132
STOP REPRODUCING VIDEO

S133
COMPUTE EVALUATION

S134
OUTPUT EVALUATION VALUE

END

# FIG.6

FROM S104

DETECT POINT OF GAZE — S105

DOES DETECTION FAIL? — S106
— YES
— NO

DETECT AREA — S107

AREA A1? — S108
— YES
— NO

AREA A2? — S117
— YES
— NO

AREA B? — S126
— YES
— NO

CNTA1: TIME OF STAY
CNTA2: TIME OF STAY
CNTB: TIME OF STAY

FLAG FA1=1? — S109
— YES
— NO

TIME OF SEARCH =TIMER T1 — S110

NUMBER OF ROUTES TO AREA A1=NUMBER OF ROUTES — S111

FLAG FA1=1 — S112

LAST AREA=AREA A1? — S113
— YES
— NO

NUMBER OF ROUTES =NUMBER OF ROUTES+1 — S114

LAST AREA =AREA A1? — S115

CNTA1=CNTA1+1 — S116

FLAG FA2=1? — S118
— YES
— NO

TIME OF SEARCH =TIMER T1 — S119

NUMBER OF ROUTES TO AREA A2=NUMBER OF ROUTES — S120

FLAG FA2=1 — S121

LAST AREA=AREA A2? — S122
— YES
— NO

NUMBER OF ROUTES =NUMBER OF ROUTES+1 — S123

LAST AREA =AREA A2 — S124

CNTA2=CNTA2+1 — S125

LAST AREA=AREA B? — S127
— YES
— NO

NUMBER OF ROUTES =NUMBER OF ROUTES+1 — S128

LAST AREA =AREA B — S129

CNTB=CNTB+1 — S130

DOES TIMER T1 COMPLETE? — S131
— NO
— YES

TO S132

21

**EP 3 970 624 B1**

**Patent documents cited in the description**

- JP 2017217051 A
- WO 2019098173 A
- JP 2017158866 A
- JP 2018140007 A